# EUROPEAN PATENT SPECIFICATION

(11) **EP 2 618 804 B1**
(45) Date of publication and mention of the grant of the patent: **11.01.2017**
(21) Application number: 11773657.9
(22) Date of filing: 20.09.2011
(51) Int. Cl.: A61K 8/34, A61K 8/35, A61K 8/41, A61K 8/49, A61Q 17/04

(54) **HIGH PROTECTION UVA/UVB COMPOSITION, ITS PREPARATION PROCESS AND TOPICAL COSMETIC COMPOSITION**
UVA/UVB-ZUSAMMENSETZUNG MIT HOHEM SCHUTZFAKTOR, HERSTELLUNGSVERFAHREN DAFÜR UND TOPISCHE KOSMETISCHE ZUSAMMENSETZUNG
COMPOSITION DE HAUTE PROTECTION CONTRE LES UVA/UVB, SON PROCÉDÉ DE PRODUCTION, ET COMPOSITION COSMÉTIQUE TOPIQUE

(30) Priority: 20.09.2010 FR 1057500
(43) Date of publication of application: 31.07.2013
(73) Proprietor: Natura Cosméticos S.A., 06882-700 ltapecerica da Serra - SP (BR)
(72) Inventor: PICONI LONGO, Ana Carolyna, 04522-32 - Morena - SP (BR); AZEVEDO TADINI, Kassandra, 13210-705 - Jundiaí- SP (BR); COLLINA ROMANHOLE, Rodrigo, 03120-010 - Mooca - SP (BR)
(74) Representative: Brady, Paul Andrew
(86) International application number: PCT/BR2011/000335
(87) International publication number: WO 2012/037626

(56) References cited:
- EP-A1- 1 997 474
- EP-A2- 1 857 096
- DE-A1- 4 440 086
- DE-A1- 19 521 951
- DE-A1-102008 033 343
- FR-A1- 2 555 898
- GB-A- 1 124 558
- JP-A- 2008 247 775

## Description

### FIELD OF THE INVENTION

The present invention refers to a fluid and transparent high protection UVA / UVB composition with superior sensory characteristics and to a process for preparing said composition.

### BACKGROUND OF THE INVENTION

It is known that the sun radiation on contact with skin may cause several types of injuries. In order to protect the skin, it is commonly used sunscreen products comprising substances capable of reflecting, scattering or absorbing photons of ultraviolet region (UV).

Among the various types of radiation emitted by the sun, there are the infrared, UVA, UVB and UVC, wherein the UV rays are the main cause of skin damage.

UVC-rays are absorbed by the higher layers of the atmosphere and stratosphere, and rarely reach the ground. With respect to UVA-rays and a portion of UVB-rays, they are transmitted through the atmosphere and, therefore, can damage the skin. As the UVB-rays have shorter wavelengths, they reach the most superficial layer of the skin - the epidermis, while UVA-rays can penetrate to the dermis - the middle layer.

Excessive exposure to the sun can result in skin burns, also known as erythema, caused mainly by UVB radiation. Over the longer term, UV radiation induces degenerative changes in cells of the skin, fibrous tissue and blood vessels leading to premature skin aging, photodermatoses, actinic keratoses and even cancer.

The sun protection factor (SPF) is a factor commonly used in sunscreens to indicate how much the product can protect the skin. SPF is a measure of how much solar energy is required to produce erythema on protected skin (i.e., in the presence of the sunscreen) relative to the amount of solar energy required to produce sunburn on unprotected skin. As the SPF value increases, sunburn protection increases. The SPF factor only indicates protection against UVB-rays, responsible for skin burns. For UVA-rays, ways of measuring and protection indicators are currently still being studied.

One of the most widely known methods to determine the protective factor for UVA is the Persistent Pigment Darkening (PPD). According to this method, the immediate pigmentation resulted from photooxidation of preexisting melanin occurring caused by UVA radiation exposure is measured. According to Colipa (The European Cosmetic Association), the recommended PPD for sunscreen products should be at least one third of the SPF.

Products for protection against the harmful rays of the sun comprise sunscreens and, according to their mechanism of action, may be classified as:
- physical filters → substances capable of reflecting and scattering UV rays, and
- chemical filters → substances capable of absorbing UV radiation and convert it into radiation with lower energy and less harmful to humans.

The damage associated with sun exposure has a cumulative effect, which contributes to the emergence of cancer many years after the exposure.

Sunscreen products may comprise only physical filters, chemical filters or a combination thereof. Sunscreen products with high amount of physical filters generally have a sticky aspect and whitish appearance after the application on the skin. Furthermore, the use of these filters makes it impossible to obtain clear compositions. Sunscreen products with high amounts of chemical filters have a high oil load, giving a oily sensation and a longer dry.

Currently, most products available at the market uses a combination of physical and chemical filters to achieve a high solar protection, primarily for obtaining a SPF higher than 50. Although they result in a product with a sensory relatively improved with the using of the said combination, the obtaining of transparent and fluid compositions with fast drying is not possible.

Document WO2007007283 discloses a sunscreen composition capable of absorbing UV radiation. This composition is liquid (gel form) and transparent.

Document US2009017081 describes a sunscreen in the form of a water-in-oil emulsion capable of preventing and inhibiting the discoloration over time.

Document DE102008033343 describes cosmetic compositions comprising neutralized 2-Phenylbenzimidazol-5-sulfonic acid.

Hence, the state of the art does not reveal a high protection UVA / UVB sunscreen composition with a high SPF and an appropriated PPD having a fluid and transparent aspect and having pleasant sensory characteristics.

The objective of the present invention is to provide a high protection UVA / UVB composition with a high SPF (greater than 50) and an appropriated PPD (at least one third of the SPF) having a fluid and transparent aspect. Furthermore, said composition has pleasant sensory characteristics, no stickiness, fast dry, transparency after the application on the skin, water and sweat resistance and high stability in storage.

In addition, a further objective of the present invention is to provide a process for preparing said high protection UVA / UVB composition.

### SUMMARY OF THE INVENTION

The present invention relates to a high protection UVA / UVB composition comprising (a) phenylbenzimidazole sulfonic acid; (b) triethanolamine; (c) an UVA chemical filter; (d) an additional chemical filter; and (e) an alcoholic carrier. A further embodiment of the present invention is the process for preparation of said composition and a topical cosmetic composition.

The composition of the present invention has the following advantages in relation to the state of art:
- high SPF (higher than 40 and more particularly in the range of about 40 to about 60)
- appropriated PPD, at least one third of the SPF;
- photostability;
- it is transparent after application;
- high fluidity;
- nice sensory characteristics;
- no stickiness;
- fast drying; and
- exclusive use of chemical filters.

### BRIEF DESCRIPTION OF THE DRAWINGS

Figure 1 shows the kinetic degradation of the high protection UVA / UVB composition of the present invention without irradiation thereof, and after 1, 2, 4 and 8 hours of exposure equivalent to natural sunlight.
Figure 2 shows the percentage decreasing in the UVA (320 to 400nm) protection for the high protection UVA / UVB protection composition of the present invention.
Figure 3 shows the graphics generated for the high protection UVA / UVB protection composition of the present invention before and after exposure to radiation in the PMMA plate.

### DETAILED DESCRIPTION OF THE INVENTION

The present invention relates to a high protection UVA / UVB composition comprising:
(a) 0.5 to 7.5% by weight of phenylbenzimidazole sulfonic acid;
(b) 2.5% by weight of triethanolamine;
(c) 6 % by weight of an UVA chemical filter;
(d) 20 to 40% by weight of an additional chemical filter;
(e) 40 to 70% by weight of an alcoholic carrier; and
(f) 2% by weight of a film former.
wherein all percentages are based on the total weight of the high protection UVA / UVB composition and no water is added to the composition.

The high protection UVA / UVB composition of the present invention can be advantageously used in cosmetic and pharmaceutical compositions for topical use.

Still, the aforementioned composition can achieve a SPF values higher than 40, more particularly in the range of about 40 to about 60, and a PPD of at least 16 without the inconvenience of high SPF compositions already know in the state of art, for example, sticky, whitish appearance in the skin after the application and the difficulty of spreading.

### Phenylbenzimidazole Sulfonic Acid

According to the invention, the phenylbenzimidazole sulfonic acid is present in said high UVA / UVB protection composition in an amount ranging from 0.5 to 7.5% by weight, based on total weight of the composition, preferably 3%.

### Triethanolamine

According to the invention, the triethanolamine is present in said high protection UVA / UVB composition, in an amount of 2.5%.

### UVA Chemical Filter

According to the invention, the UVA chemical filter is present in said high protection UVA / UVB composition, in an amount of 6%.

The chemical UVA filter is selected from the group comprising: butyl methozydibenzoylmethane, diethylamino hydroxybenzoyl hexyl benzoate, bis-ethylhexyloxyphenol methoxyphenyl triazine, disodium phenyl dibenzimidazole tetrasulfonate, terephthalylidene dicamphor sulfonic acid and mixture thereof.

According to a preferred embodiment of the invention, the UVA chemical filter is selected from the group comprising: butyl methoxydibenzoylmethane, diethylamino hydroxybenzoyl hexyl benzoate and mixture thereof.

### Additional Chemical Filter

The additional chemical filter is selected from the group comprising: benzophenone-3, octocrylene, homosalate, ethylhexyl salicylate, butyl methoxydibenzoylmethane, diethylamino hydroxybenzoyl hexyl benzoate, bis-ethylhexyloxyphenol methoxyphenyl triazine, drometrizole trisiloxane, 4-methylbenzylidene camphor, benzophenone-4, diethylhexyl butamido triazone, ethylhexyl methoxycinnamate, ethylhexyl triazone, ethylhexyl dimethyl paba, homomenthyl salicylate, isoamyl p-methoxycinnamate, polysilicone-15 and mixture thereof.

According to the invention, the additional chemical filter is present in said high protection UVA / UVB composition, in an amount ranging from 20 to 40% by weight, based on total weight of the composition, preferably 29%.

### Alcoholic Carrier

The alcoholic carrier used in a preferred embodiment of the present invention presents water content not more than 4%, by weight based on total weight of said carrier.

An even more preferred embodiment of the present invention, the alcoholic carrier present in said high protection UVA / UVB composition, in an amount ranging from 50 to 55% by weight, based on total weight of the composition.

### Film Former

The film former is used in the present composition in an amount of 2% by weight, based on total weight of the composition.

### Other Optional Components

The high protection UVA / UVB composition of the present invention further comprises optional components commonly used in cosmetics or pharmaceuticals in accordance with the intended application for the composition being prepared.

Among the optional components, it can be cited: antioxidant, preservative, support microcrystalline network former, polymeric agent, copolymeric agent, sensory modifier, cosolvent, denaturing agent, consistency agent, emollient, humectant, moisturizing agent, constraint agent and mixture thereof. Preferably, the sensory modifier used is a silicone, more preferably cyclomethicone.

The phenylbenzimidazole sulfonic acid is used in the present invention since it promotes a high UVB protection. However, since the phenylbenzimidazole sulfonic acid is water soluble and there is no addition of water in the high UVA / UVB protection composition (almost anhydrous) this filter would not solubilized in the composition.

Thus, in order to solve this technical problem, another embodiment of the present invention is a process of preparing a high UVA / UVB protection composition, as defined above, comprising the following steps:
**(i)** solubilize triethanolamine in alcoholic carrier, and
**(ii)** when stirring, add phenylbenzimidazole sulfonic acid to the mixture obtained in step *(i)*;
**(iii)** in parallel, heat the additional chemical filters at a temperature ranging from 50 to 70°C, preferably 60°C;
**(iv)** remove from the heating the mixture obtained in step (iii) and when it is at a temperature ranging from *30 to 50°C, preferably 40°C, add it to the mixture obtained in step **(ii)**, and
**(v)** stir to obtain the final composition.

### EXAMPLE

The present invention will be illustrated through an example in order to better describe it and demonstrate the effectiveness of the high protection UVA / UVB composition described in this patent application. However, this example is merely illustrative of a preferred embodiment of the present invention, therefore, should not be taken as limiting the scope of this patent application.

**Table 1: Components of the high protection UVA / UVB composition**

| Component | Content (%) |
|---|---|
| Film former | 2.00 |
| Additional Chemical Filter | 26.00 |
| UVA filter | 6.00 |
| Phenylbenzimidazole sulfonic acid | 3.00 |
| Triethanolamine | 2.50 |
| Alcoholic carrier | qsp 100.00 |

This high protection UVA / UVB sunscreen composition with a high SPF and an appropriated PPD having a fluid and transparent aspect and having pleasant sensory characteristics

### Evaluation of skin acceptability in adults

Based on the inclusion and exclusion criteria, 33 volunteers of both sexes with age ranging from 18 to 60 years and phototype (Fitzpatrick) of I to IV were selected to participate in the evaluation.

The volunteers were initially analyzed by a dermatologist and a oculist in order to check the inclusion and exclusion criteria and for a general dermatologic and ophthalmologic analyses, the data were completed in the clinical record.

After the analyses, samples of the high protection UVA / UVB composition were distributed together with guidelines defining the use mode. A journal was given so that the volunteer fill out it and give it back at the end of the study.

New dermatologic and ophthalmologic analyses were performed with 21 ± 2 days of study, the data were duly filled in clinic records. The samples of the high protection UVA / UVB composition were weighed at the beginning and the end of study

From the 33 volunteers, 31 completed the study, since 02 volunteers did not return for the final analysis due to personal reasons unrelated to the study.

Of the 31 volunteers that completed the study showed, no one showed reaction in the skin, ocular or periocular area under dermatological and ophthalmological monitoring. The final results of the dermatologic evaluation are presented in Table 2.

**Table 2: Final data of dermatologic analysis.**

| | **Erythema** | **Edema** | **Dryness** | **Total** | **Classification** | **Reaction Extension** |
|---|---|---|---|---|---|---|
| **01** | 0 | 0 | 0 | D | NR | - |
| **02** | 0 | 0 | 0 | 0 | NR | - |
| **03** | 0 | 0 | 0 | 0 | NR | - |
| **04** | 0 | 0 | 0 | 0 | NR | - |
| **05** | 0 | 0 | 0 | 0 | NR | - |
| **06** | 0 | 0 | 0 | 0 | NR | - |
| **07** | 0 | 0 | 0 | 0 | NR | - |
| **08** | 0 | 0 | 0 | 0 | NR | - |
| **09** | 0 | 0 | 0 | 0 | NR | - |
| **10** | 0 | 0 | 0 | 0 | SR | - |
| **11** | | | | | | |
| **12** | 0 | 0 | 0 | 0 | NR | - |
| **13** | 0 | 0 | 0 | 0 | NR | - |
| **14** | 0 | 0 | 0 | 0 | NR | - |
| **15** | 0 | 0 | 0 | 0 | NR | - |
| **16** | 0 | 0 | 0 | 0 | NR | - |
| **17** | 0 | 0 | 0 | 0 | NR | - |
| **18** | 0 | 0 | 0 | 0 | NR | - |
| **19** | 0 | 0 | 0 | 0 | NR | - |
| **20** | 0 | 0 | 0 | 0 | NR | - |
| **21** | 0 | 0 | 0 | 0 | NR | - |
| **22** | 0 | 0 | 0 | 0 | NR | - |
| **23** | 0 | 0 | 0 | 0 | NR | - |
| **24** | 0 | 0 | 0 | 0 | NR | - |
| **25** | 0 | 0 | 0 | 0 | NR | - |
| **26** | 0 | 0 | 0 | 0 | NR | - |
| **27** | | | | | | |
| **28** | 0 | 0 | 0 | 0 | NR | - |
| **29** | 0 | 0 | 0 | 0 | NR | - |
| **30** | 0 | 0 | 0 | 0 | NR | - |
| **31** | 0 | 0 | 0 | 0 | NR | - |
| **32** | 0 | 0 | 0 | 0 | NR | - |
| **33** | 0 | 0 | 0 | 0 | NR | - |

In the above table, "0" means no erythema, no edema and no scaling and "NR" means no reaction.

Table 3 shows the data from the final ophthalmic analysis of the volunteers.

**Table 3: Data of the final ophthalmic analysis.**

| **N° Vol.** | **Blepharitis** | **Meibomitis** | **Hyperemia** | **Lachrymation** | **Chemosis** | **Cornea** |
|---|---|---|---|---|---|---|
| **01** | 0 | 0 | 0 | 0 | 0 | No modification |
| **02** | 0 | 0 | 0 | 0 | 0 | No modification |
| **03** | 0 | 0 | 0 | 0 | 0 | No modification |
| **04** | 0 | 0 | 0 | 0 | 0 | No modification |
| **05** | 0 | 0 | 0 | 0 | 0 | No modification |
| **06** | 0 | 0 | 0 | 0 | 0 | No modification |
| **07** | 0 | 0 | 0 | 0 | 0 | No modification |
| **08** | 0 | 0 | 0 | 0 | 0 | No modification |
| **09** | 0 | 0 | 0 | 0 | 0 | No modification |
| **10** | 0 | 0 | 0 | 0 | 0 | No modification |
| **11** | | | | | | |
| **12** | 0 | 0 | 0 | 0 | 0 | No modification |
| **13** | 0 | 0 | 0 | 0 | 0 | No modification |
| **14** | 0 | 0 | 0 | 0 | 0 | No modification |
| **15** | 0 | 0 | 0 | 0 | 0 | No modification |
| **16** | 0 | 0 | 0 | 0 | 0 | No modification |
| **17** | 0 | 0 | 0 | 0 | 0 | No modification |
| **18** | 0 | 0 | 0 | 0 | 0 | No modification |
| **19** | 0 | 0 | 0 | 0 | 0 | No modification |
| **20** | 0 | 0 | 0 | 0 | 0 | No modification |
| **21** | 0 | 0 | 0 | 0 | 0 | No modification |
| **22** | 0 | 0 | 0 | 0 | 0 | No modification |
| **23** | 0 | 0 | 0 | 0 | 0 | No modification |
| **24** | 0 | 0 | 0 | 0 | 0 | No modification |
| **25** | 0 | 0 | 0 | 0 | 0 | No modification |
| **26** | 0 | 0 | 0 | 0 | 0 | No modification |
| **27** | | | | | | |
| **28** | 0 | 0 | 0 | 0 | 0 | No modification |
| **29** | 0 | 0 | 0 | 0 | 0 | No modification |
| **30** | 0 | 0 | 0 | 0 | 0 | No modification |
| **31** | 0 | 0 | 0 | 0 | 0 | No modification |
| **32** | 0 | 0 | 0 | 0 | 0 | No modification |
| **33** | 0 | 0 | 0 | 0 | 0 | No modification |

In the above table, "0" means lack of reactions.

Table 4 presents the discomfort feelings reported by the volunteers.

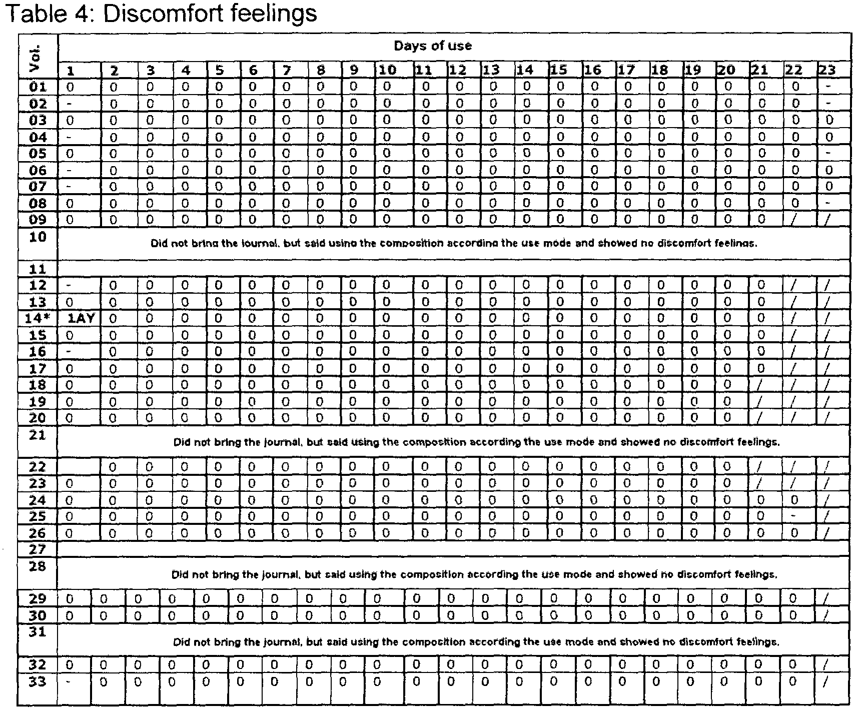

In the above table, "0" means no discomfort feeling; "-" means volunteer did not use the high protection UVA/UVB composition; and "/" means volunteer has already completed the study.

From the above results, it can be concluded that the high protection UVA / UVB of the present invention is safe for human use and any irritation or sensitization reactions causing were caused.

### Evaluation of comedogenic potential

Based on the inclusion and exclusion criteria, 38 volunteers of both sexes with age ranging from 18 to 40 years and phototype (Fitzpatrick) of I to IV were selected to participate in the evaluation.

Samples of the high protection UVA / UVB composition were distributed together with guidelines defining the use mode for 4 weeks. A journal was given so that the volunteer fill out it and give it back at the end of the study.

At the beginning (D0) and at the end (D28) of the study, the volunteers were analyzed by a dermatologist in order to check possible adverse events. The regions analyzed were right or left malar; chin; and right and left forehead.

An exploratory data analysis were performed. The times were compared using a non-parametric Wilcoxon signed rank. The confidence level considered was of 95%. The software used was XSLTAT 2009 and STATA 10.

Of the 38 volunteers, 03 volunteers (008, 012 and 013) were excluded due to a selection failure and 04 volunteers (005, 006, 015 and 027) did not concluded the research due to personal reasons unrelated to the study.

Table 5 shows the number of injuries by volunteers (the volunteers that did not show no lesions were not included in the table).

**Table 5: Total number of lesions detected in all areas analyzed by volunteer at D0 and D28.**

| **Volunteer** | **D0** | **D28** | **Δ(D28-D0)** |
|---|---|---|---|
| 001 | 5 | 14 | 9 |
| 002 | 5 | 3 | -2 |
| 003 | 20 | 10 | -10 |
| 004 | 4 | 4 | 0 |
| 007 | 8 | 4 | -4 |
| 009 | 18 | 25 | 7 |
| 010 | 22 | 24 | 2 |
| 011 | 5 | 9 | 4 |
| 014 | 4 | 0 | -4 |
| 016 | 2 | 3 | 1 |
| 017 | 7 | 5 | -2 |
| 018 | 13 | 5 | -8 |
| 019 | 2 | 0 | -2 |
| 020 | 2 | 0 | -2 |
| 021 | 6 | 3 | -3 |
| 022 | 5 | 6 | 1 |
| 023 | 4 | 9 | 5 |
| 024 | 9 | 0 | -9 |
| 025 | 2 | 1 | -1 |
| 026 | 2 | 2 | 0 |
| 028 | 9 | 0 | -9 |
| 029 | 6 | 1 | -5 |
| 030 | 18 | 2 | -16 |
| 031 | 5 | 3 | -2 |
| 032 | 7 | 0 | -7 |
| 033 | 8 | 0 | -8 |
| 034 | 15 | 4 | -11 |
| 035 | 37 | 19 | -18 |
| 036 | 10 | 1 | -9 |
| 037 | 1 | 0 | -1 |
| 038 | 5 | 2 | -3 |
| **Average** | **8,58** | **5,13** | **-3,45** |
| **Median** | 6,0 | 3,0 | -2,0 |
| **Minimum** | 1,0 | 0,0 | -18,0 |
| **Maximium** | 37,0 | 25,0 | 9,0 |
| **EP** | 1,4 | 1,2 | 1,1 |
| IC 95% | 2,8 | 2.4 | 2,2 |
| **Δ(%) regarding D0** | | | **-40,2** |
| **% volunteers with positive effect** | | | **71,0** |
| **P-Value** | | | **0,0038**** |

| | | | |
|---|---|---|---|
| *** Significant level of 0.1% | | | |

In view of the above results, it can be concluded that the high protection UVA / UVB composition of the present invention presents no comedogenic potential.

### Evaluation of photoallergy and phototoxicity in the skin

Based on the inclusion and exclusion criteria, 42 volunteers of both sexes with age ranging from 18 to 70 years and phototype (Fitzpatrick) of II and III were selected to participate in the evaluation.

### Photoallergy

A sample was applied always in the same location (right or left dorsal area), properly protected, twice a week for three weeks (total of six applications). The patch test was removed after 24 hours of the application and the area were immediately evaluated and irradiated (12-15 joules) with UVA and UVB light. The adjacent area of application and the point of contact patch with sterile saline solution were irradiated and used as control. After the applications and irradiations, a rest period of at least ten days when no patch and no irradiation were performed occurred.

Then, a patch of the sample was applied in a location where no patch has been placed yet. Tests were removed after 24 hours of application and the test areas were irradiated with UVA light. The adjacent area of application and the point of contact patch with sterile saline solution were irradiated and used as control. The volunteers were instructed to protect from the sun the irradiated skin area. Evaluations were performed after the irradiation and after 24, 48 and 72 hours of the last irradiation.

### Phototoxicity

A sample was applied once, properly protected. The patch test was removed after 24 hours of the application and the area were immediately evaluated and irradiated (12-15 joules) with UVA light. The adjacent area of application and the point of contact patch with sterile saline solution were irradiated and used as control. The volunteers were instructed to protect from the sun the irradiated skin area. Evaluations were performed after the irradiation and after 24, 48 and 72 hours of the last irradiation.

Of the 42 volunteers, 07 volunteers (208, 210, 214, 215, 216, 217 and 232) were excluded in view of a selection failure (SF) and 08 volunteers (201, 209, 213, 223, 224, 237, 239 and 242) withdrew from the study due to personal reasons unrelated to the study. A volunteer (241) showed a reaction in previously sensitized skin, individual pre-disposing, due to skin exposure to the tape, so it was removed from the search. Thus, 26 volunteers completed the study.

No adverse reactions (erythema, edema, papules or vesicles) have been detected in the applications and control areas during, as showed in the following tables.

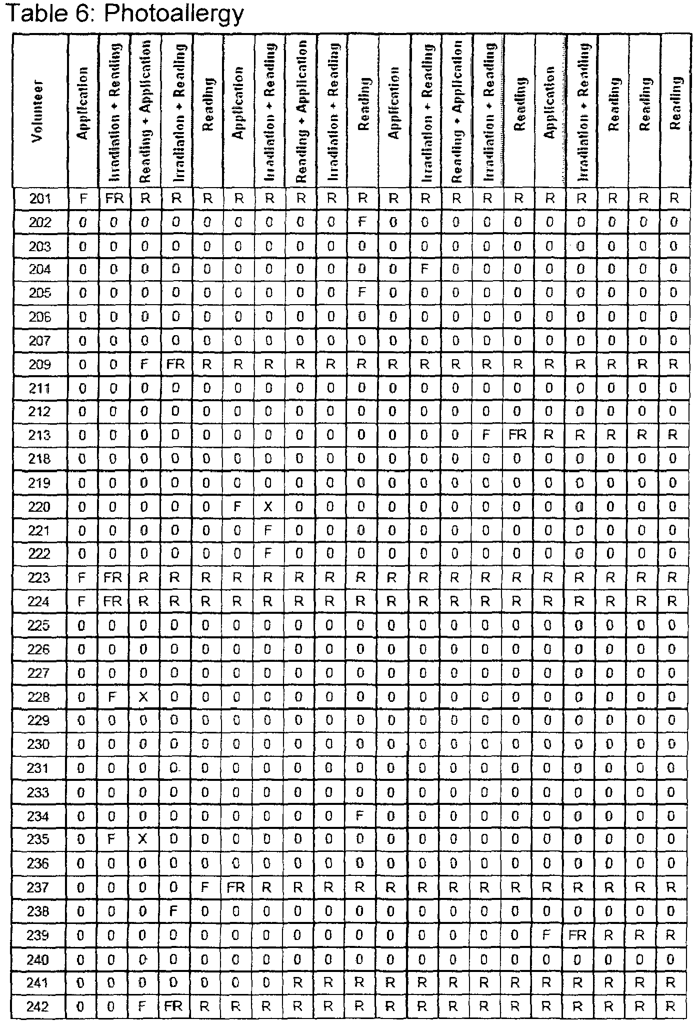

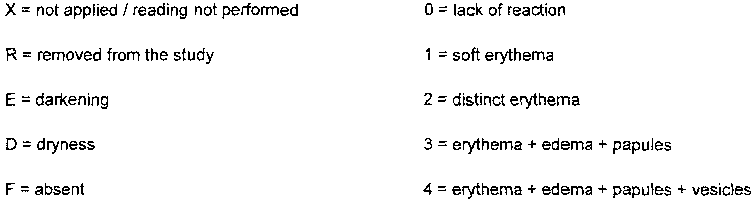

**Tabela 7: Phototoxicity**

| **Application-Reading** | | | | | |
|---|---|---|---|---|---|
| **Volunteer** | **Application** | **Irradiation** + **Reading** | **Reading 24 h** | **Reading 48 h** | **Reading 72 h** |
| 201 | FR | R | R | R | R |
| 202 | 0 | 0 | 0 | 0 | 0 |
| 203 | 0 | 0 | 0 | 0 | 0 |
| 204 | 0 | 0 | 0 | 0 | 0 |
| 205 | 0 | 0 | 0 | 0 | 0 |
| 206 | 0 | 0 | 0 | 0 | 0 |
| 207 | 0 | 0 | 0 | 0 | 0 |
| 209 | FR | R | R | R | R |
| 211 | 0 | 0 | 0 | 0 | 0 |
| 212 | 0 | 0 | 0 | 0 | 0 |
| 213 | FR | R | R | R | R |
| 218 | 0 | 0 | 0 | 0 | 0 |
| 219 | 0 | 0 | 0 | 0 | 0 |
| 220 | 0 | 0 | 0 | 0 | 0 |
| 221 | 0 | 0 | 0 | 0 | 0 |
| 222 | 0 | 0 | 0 | 0 | 0 |
| 223 | FR | R | R | R | R |
| 224 | FR | R | R | R | R |
| 225 | 0 | 0 | 0 | 0 | 0 |
| 226 | 0 | 0 | 0 | 0 | 0 |
| 227 | 0 | 0 | 0 | 0 | 0 |
| 228 | 0 | 0 | 0 | 0 | 0 |
| 229 | 0 | 0 | 0 | 0 | 0 |
| 230 | 0 | 0 | 0 | 0 | 0 |
| 231 | 0 | 0 | 0 | 0 | 0 |
| 233 | 0 | 0 | 0 | 0 | 0 |
| 234 | 0 | 0 | 0 | 0 | 0 |
| 235 | 0 | 0 | 0 | 0 | 0 |
| 236 | 0 | 0 | 0 | 0 | 0 |
| 237 | FR | R | R | R | R |
| 238 | 0 | 0 | 0 | 0 | 0 |
| 239 | FR | R | R | R | R |
| 240 | 0 | 0 | 0 | 0 | 0 |
| 241 | R | R | R | R | R |
| 242 | FR | R | R | R | R |

| | | | | | |
|---|---|---|---|---|---|
| X = not applied / reading not performed 0 = lack of reaction R = removed from the study 1 = soft erythema E = darkening 2 = distinct erythema D = dryness 3 = erythema + edema + papules F = absent 4 = erythema + edema + papules + vesicles | | | | | |

From the above results, it can be concluded that the high protection UVA / UVB composition of the present invention does not provokes photoallergy and phototoxicity.

### Evaluation of irritant and allergen potential

Based on the inclusion and exclusion criteria, 72 volunteers of both sexes with age ranging from 18 to 70 years and phototype (Fitzpatrick) of I to IV were selected to participate in the evaluation.

The volunteers were submitted to a general dermatologic analysis by a dermatologist and were received instructions regarding the study.

The study consisted of maximized and controlled applications of the high protection UVA / UVB composition followed by successive readings for six weeks. The composition was applied always in the same dorsal region of the volunteer under semiocclusive dressings. A dressing with saline was used as negative control.

The applications were repeated nine times (D1, D3, D5, D8, D10, D12, D15, D17 and D19) in a period of three consecutive weeks, on alternate days, this period was necessary for a possible induction of immune response or a possible appearance of signs or irritating sensation. The dressing was removed approximately 48 hours after each application, except on weekends wherein the dressings remained in contact with the skin for 72 hours.

To perform all technical evaluations, the excess material were removed with a sterile saline soaked-cotton and waited for a period of at least 30 minutes for the reading. After this induction period, the volunteers began a rest period of two weeks, in which no dressing was applied.

Then, in D36, a single application of the composition was performed at the area where it was performed the induction period and another application on virgin area for a period of 48 hours in order to prove a possible allergic process induced - phase challenge. The dressing was removed after 48 hours of contact (D38) with the skin and the evaluations about 30 minutes and 24 hours after its removal (D39).

A dressing with a saline solution was used as control in all applications of the induction phase and single phase implementation challenge both the virgin area and in the initial area.

Of the 72 volunteers, 04 volunteers (121, 151, 157 e 171) were excluded in view of a selection failure (SF) and 18 volunteers (105, 110, 116, 117, 118, 129, 131, 134, 136, 146, 148, 150, 153, 156, 158, 164, 166 e 169) withdrew from the study due to personal reasons unrelated to the study. Thus, 50 volunteers completed the study.

No adverse reactions (erythema, edema, papules or vesicles) have been detected in the applications and control areas during, as showed in Table 8.

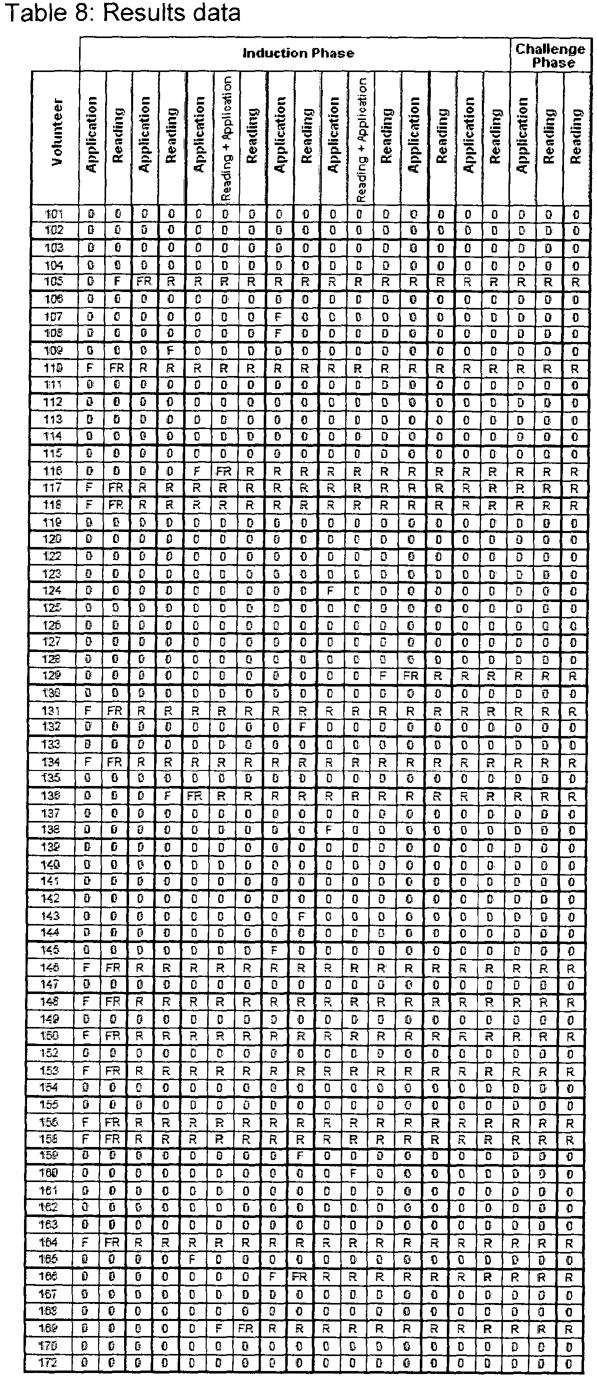

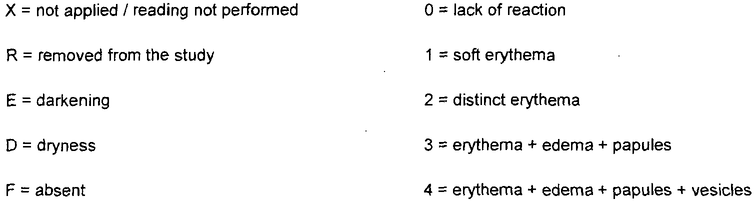

In view of the above results, it can be concluded that the high protection UVA / UVB composition of the present invention does not induces irritation and sensitization process in the skin.

### Evaluation of skin tolerance

Based on the inclusion and exclusion criteria, 10 volunteers of both sexes with age ranging from 18 to 60 years and phototype (Fitzpatrick) of I to IV were selected to participate in the evaluation.

The high protection UVA / UVB composition was applied in the "elbow bending" (in this region, the skin is thin and reactive) twice daily for four consecutive days and once on the fifth day. After the first application of each day, both forearms were flexed on the arm for about 15 minutes. At the end of time, the forearms were extended for about 30 minutes. The area was rinsed with running water.

The clinical examination of the experimental area and a interview with the volunteers were performed by the person responsible by the study. The examination was performed visually under standard "daylight" before application and 45 ± 5 minutes after each application. At the same time, each volunteer was questioned about any possible discomfort feelings. The results are show in Table 9.

**Table 9: Results data**

| **Clinical Manifestations** | **Volunteers Presenting Clinical Manifestations** | **Discomfort Feelings** | **Volunteers Presenting Discomfort Feelings** |
|---|---|---|---|
| None | 0% | None | 0% |

In view of the above results, it can be concluded that the high protection UVA / UVB composition of the present invention has a very good skin compatibility.

### Evaluation of photostability

Small drops of the high protection UVA / UVB composition was applied to the substrate over the entire plate of PMMA - poly(methyl methacrylate). A plate of PMMA with dispersed glycerin was used to record the baseline of the spectrophotometer.

Then the composition drops was quickly spread over the entire surface of the PMMA plate to obtain a visually uniform film. Then a standard drying process consisting in maintaining the composition applied on the substrate for a period of 15 minutes under controlled temperature at 20°C ± 3 °C was performed.

The samples were taken to Suntest CPS+ solar simulator with a fixed irradiance level of 750W/m², a pre-set exposure time and a controlled temperature of the exposing chamber (not more than 40°C).

In Labsphere UV1000S spectrophotometer, absorbance readings were performed at four positions in the sample initially established according to the exposure times of 12, 24, 48 and 96 minutes in Suntest CPS+ solar simulator with an irradiance of 750W/m².

Through the natural radiation conditions, the periods of simulated irradiation of 12, 24, 48 and 96 minutes correspond to 1, 2, 4 and 8 hours of natural sunlight exposure, respectively. The decreasing kinetic in the UV absorption levels were used to evaluate the photostability of the high protection UVA / UVB composition.

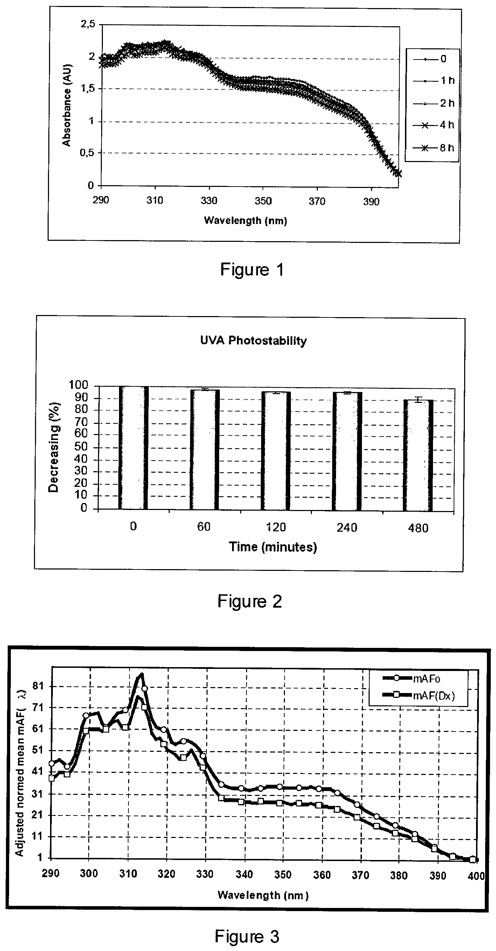

The results of the present study are shown in Figure 1 and Figure 2. Through the analysis thereof, it is observed that the maximum percentage decays obtained (after 8 hours of natural sunlight) were 9.24% in the UVA region, hence the high protection UVA / UVB composition shows high photostability in the UVA region.

### Evaluation of sun protection factor

Based on the inclusion and exclusion criteria, 10 volunteers of both sexes with phototype (Fitzpatrick) of I to III were selected to participate in the evaluation.

The area tested was the back between the beltline and the scapulae (shoulder blades) and lateral to midline. Two test site areas were used: one for before water immersion and one for after 120 minutes of water immersion (4 procedures of 20 minutes of immersion in moderately agitated water followed by a 15 minute rest), then the test sites were air dried for 15 minutes without toweling. One additional test site area was used for the P3 control. After applying the composition, a waiting period of 15 to 30 minutes was employed.

A series of UV light exposures was administered to the subsites on each subject with the Modified Solar Ultraviolet Simulator, Model 10S, (the source of illumination for the test site scoring was a warm white fluorescent light which provided a level of 450-550 lux). One series of exposures were administered to the untreated, unprotected skin to determine the MED (minimal erythema dose). The protected test sites were also exposed to UV light. The standard time intervals selected were a geometric series represented by (1.25)n and (1.12)n.

No adverse reactions nor adverse events were observed in any of the subjects, as shown in Table 11. Static SPF (95% Cl) = 62.88 (60.22-65.54) / VWR (Very Water Resistance) SPF (95% Cl) = 60.07 (57.77-62.37).

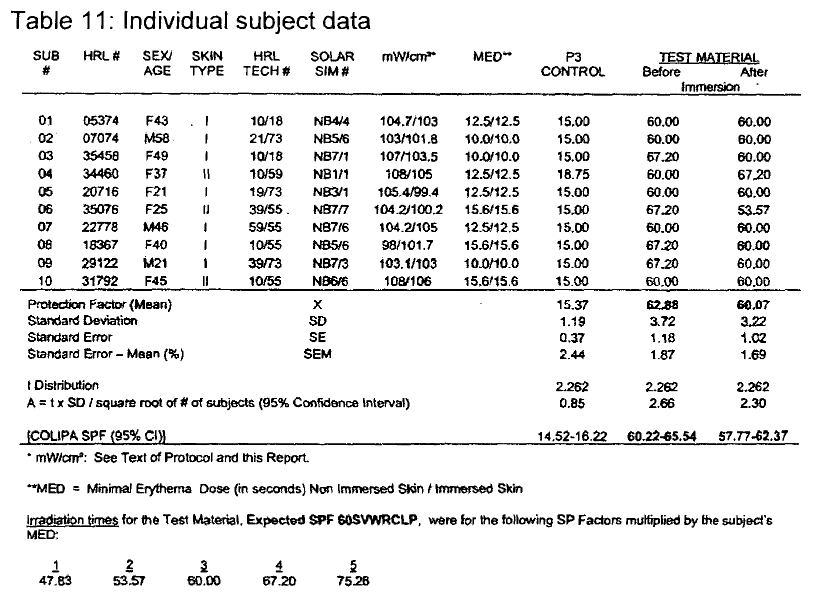

### Evaluation of UVA protection

The present evaluation is based on the measurement of UV transmittance through the sample that is applied to a suitable substrate and with controlled roughness. The composition is exposed to a radiation dosage proportional to the initial UVA Protection Factor (FPUVA₀), calculated by using the transmittance data of a non-exposed sample. The final UVA Protection Factor (FPUVA) is calculated by using the transmittance data adjusted of the sample exposed to UV radiation.

Small drops of the high protection UVA / UVB composition was applied to the substrate over the entire plate of PMMA - poly(methyl methacrylate). A plate of PMMA with dispersed glycerin was used to record the baseline of the spectrophotometer.

Then the composition drops was quickly spread over the entire surface of the PMMA plate to obtain a visually uniform film. Then a standard drying process consisting in maintaining the composition applied on the substrate for a period of 15 minutes under controlled temperature at 20°C ± 3°C was performed.

Each PMMA plate was measured at four different areas to ensure that at least 2 cm² are evaluated. The FPUVA₀ and FPUVA of a plate are calculated by using the absorbance average of all areas of the same PMMA plate. As result acceptance criteria, it was stipulated that if the coefficient of variation for the absorbance between the different areas exceeds 50%, then this PMMA plate must be rejected and a new plate should be prepared.

The FPUVA₀ and FPUVA of the composition is the average of FPUVA₀ and FPUVA of at least three individual plates. As result acceptance criteria, it was stipulated that if the coefficient of variation between FPUVA₀ and FPUVA of individual plates exceeds 20% then new plates should be prepared.

The conditions of the present study were described in Table 12.

**Table 10: Study conditions**

| SPF in vivo | 62,88 |
|---|---|
| SP-UVAo | 26,77 |
| Adjustment coefficient for SPFo = SPFn | 0,76 |
| Dx (with Do by SP-UVAo unity:) | 32,12 |
| Irradiance(W.m-²) | 750 |
| UV exposure (Suntest) | 01:07 |
| *SP-UVA in vitro 2007* Colipa | 21,49 |

The high protection UVA / UVB composition spectrum before and after radiation exposure are shown in Figure 3. The SPF value of 62.88 (resulted of an *in vivo* test) was used for calculating the UVA protection. Therefore, the composition of the present invention has UVA protection effectiveness, since the average UVA protection values are 21,49.

### Evaluation of stability

Aparência: As amostras submetidas às condições de 45°C, 37°C, 5°C e 25°C por 3 meses não apresentaram variações significativas.

Cor: As amostras submetidas às condições de 45°C, 37°C, 5°C e 25°C por 3 meses não apresentaram variações significativas.

Odor: As amostras submetidas às condições de 45°C, 37°C, 5°C e 25°C por 3 meses não apresentaram variações significativas.

In this study, the high protection UVA / UVB composition was submitted under accelerated aging conditions (45°C, 37°C and 5°C and 25°C for 3 months) in order to evaluate the following parameters:
- Physicochemical Parameter:
   Viscosity: the samples submitted to the accelerated aging conditions kept the viscosity within the specified range.
- Organoleptic parameters:
   Appearance: the samples submitted to the accelerated aging conditions showed no significant variations.
   Color: The samples submitted to the accelerated aging conditions showed no significant variations.
   Odor: Samples submitted to the accelerated aging conditions s showed no significant variations.

## Claims

1. High protection UVA/UVB cosmetic composition, **characterized by** comprising:
(a) 0.5 to 7.5% by weight of phenylbenzimidazole sulfonic acid;
(b) 2.5% by weight of triethanolamine;
(c) 6% by weight of an UVA chemical filter;
(d) 20 to 40% by weight of an additional chemical filter;
(e) 40 to 70% by weight of an alcoholic carrier; and
(f) 2% by weight of a film former.
wherein all percentages are based on the total weight of the high protection UVA / UVB composition and no water is added to the composition.

2. Composition, according to claim 1, **characterized in that** the phenylbenzimidazole sulfonic acid is present in an amount ranging from 1 to 5%, by weight, based on total weight of the composition.

3. Composition, according to claim 2, **characterized in that** the phenylbenzimidazole sulfonic acid is present in an amount of 3%, by weight, based on total weight of the composition.

4. Composition, according to any one of claims 1 to 3, **characterized in that** the chemical UVA filter is selected from the group comprising: butyl methoxydibenzoylmethane, diethylamino hydroxybenzoyl hexyl benzoate, bis-ethylhexyloxyphenol methoxyphenyl triazine, disodium phenyl dibenzimidazole tetrasulfonate, terephthalylidene dicamphor sulfonic acid and mixture thereof.

5. Composition, according to claim 4, **characterized in that** the UVA chemical filter is selected from the group comprising: butyl methoxydibenzoylmethane, diethylamino hydroxybenzoyl hexyl benzoate and mixture thereof.

6. Composition, according to claims 1 to 5, **characterized in that** the additional chemical filter is selected from the group comprising: benzophenone-3, octocrylene, homosalate, ethylhexyl salicylate, butyl methoxydibenzoylmethane, diethylamino hydroxybenzoyl hexyl benzoate, bis-ethylhexyloxyphenol methoxyphenyl triazine, drometrizole trisiloxane, 4-methylbenzylidene camphor, benzophenone-4, diethylhexyl butamido triazone, ethylhexyl methoxycinnamate, ethylhexyl triazone, ethylhexyl dimethyl paba, homomenthyl salicylate, isoamyl p-methoxycinnamate, polysilicone-15 and mixture thereof.

7. Composition, according to any one of claims 1 to 6, **characterized in that** the alcoholic carrier presents water content not more than 4%, by weight based on total weight of said carrier.

8. Composition, according to any one of claims 1 to 7, **characterized in that** the alcoholic carrier present in said high protection UVA / UVB composition, in an amount ranging from 50 to 55% by weight, based on total weight of the composition.

9. Composition, according to claims 1 to 8, **characterized in that** it comprises optional components selected from the group comprising antioxidant, preservative, support microcrystalline network former, polymeric agent, copolymeric agent, sensory modifier, cosolvent, denaturing agent, consistency agent, emollient, humectant, moisturizing agent, constraint agent and mixture thereof.

10. Composition, according to claims 1 to 9, **characterized in that** the additional chemical filter is present in of 29% by weight, based on total weight of the composition.

11. Process for preparing a high protection UVA/UVB composition, as define in any one of claims 1 to 10, **characterized by** comprising the following steps:
(i) solubilize triethanolamine in alcoholic carrier, and
(ii) when stirring, add phenylbenzimidazole sulfonic acid to the mixture obtained in step *(i)*;
(iii) in parallel, heat the chemical filters at a temperature ranging from 50 to 70°C, preferably 60°C;
(iv) remove from the heating the mixture obtained in step (iii) and when it is at a temperature ranging from 30 to 50°C, preferably 40°C, add it to the mixture obtained in step (ii), and
(v) stir.

## Patentansprüche

1. Kosmetische UVA/UVB-Zusammensetzung mit hohem Schutzfaktor, **dadurch gekennzeichnet, dass** sie umfasst:
(a) 0,5 bis 7,5 Gewichts-% Phenylbenzimidazolsulfonsäure,
(b) 2,5 Gewichts-% Triethanolamin,
(c) 6 Gewichts-% eines chemischen UVA-Filters,
(d) 20 bis 40 Gewichts-% eines zusätzlichen chemischen Filters,
(e) 40 bis 70 Gewichts-% eines alkoholischen Trägers und
(f) 2 Gewichts-% eines Filmbildners,
worin alle Prozentgehalten auf dem Gesamtgewicht der UVA/UVB-Zusammensetzung mit hohem Schutzfaktor beruhen und zu der Zusammensetzung kein Wasser gegeben ist.

2. Zusammensetzung nach Anspruch 1, **dadurch gekennzeichnet, dass** die Phenylbenzimidazolsulfulonsäure in einer Menge von 1 bis 5 Gewichts-%, bezogen auf das Gesamtgewicht der Zusammensetzung, vorliegt.

3. Zusammensetzung nach Anspruch 2, **dadurch gekennzeichnet, dass** die Phenylbenzimidazolsulfonsäure in einer Menge von 3 Gewichts-%, bezogen auf das Gesamtgewicht der Zusammensetzung, vorliegt.

4. Zusammensetzung nach irgendeinem der Ansprüche 1 bis 3, **dadurch gekennzeichnet, dass** der chemische UVA-Filter aus der Butylmethoxydibenzoylmethan, Diethylaminohydroxybenzoylhexylbenzoat, Bis-ethylhexyloxyphenolmethoxyphenyltriazin, Dinatriumphenyldibenzimidazoltetrasulfonat, Terephthalylidendikampfersulfonsäure und Mischungen davon umfassenden Gruppe ausgewählt ist.

5. Zusammensetzung nach Anspruch 4, **dadurch gekennzeichnet, dass** der chemische UVA-Filter aus der Butylmethoxydibenzoylmethan, Diethylaminohydroxybenzoylhexylbenzoat und Mischungen davon umfassenden Gruppe ausgewählt ist.

6. Zusammensetzung nach den Ansprüchen 1 bis 5, **dadurch gekennzeichnet, dass** der zusätzliche chemische Filter aus der Benzophenon-3, Octocrylen, Homosalat, Ethylhexylsalicylat, Butylmethoxydibenzoylmethan, Diethylaminohydroxybenzoylhexylbenzoat, Bis-ethylhexyloxyphenolmethoxy-phenyltriazin, Drometrizoltrisiloxan, 4-Methylbenzylidenkampfer, Benzophenon-4, Diethylhexylbutamidotriazon, Ethylhexylmethoxycinnamat, Ethylhexyltriazon, Ethylhexyldimethyl-paba, Homomenthylsalicylat, Isoamyl-p-methoxycinnamat, Polysilikon-15 und Mischungen davon umfassenden Gruppe ausgewählt ist.

7. Zusammensetzung nach irgendeinem der Ansprüche 1 bis 6, **dadurch gekennzeichnet, dass** der alkoholische Träger einen Wassergehalt von nicht mehr als 4 Gewichts-%, bezogen auf das Gesamtgewicht des Trägers, aufweist.

8. Zusammensetzung nach irgendeinem der Ansprüche 1 bis 7, **dadurch gekennzeichnet, dass** der alkoholische Träger in der UVA/UVB-Zusammensetzung hohen Schutzfaktors in einer Menge von 50 bis 55 Gewichts-%, bezogen auf das Gesamtgewicht der Zusammensetzung, vorliegt.

9. Zusammensetzung nach den Ansprüchen 1 bis 8, **dadurch gekennzeichnet, dass** sie optionale Bestandteile enthält, die aus der Antioxidationsmittel, Konservierungsmittel, unterstützende mikrokristalline Netzwerkbildner, polymeres Mittel, copolymeres Mittel, sensorisches Modifizierungsmittel, Cosolvent, Denaturierungsmittel, Konsistenzmittel, Emollient, Feuchthaltemittel, feuchtigkeitsspendendes Mittel, Beschränkungsmittel und Mischungen davon umfassenden Gruppe ausgewählt ist.

10. Zusammensetzung nach den Ansprüchen 1 bis 9, **dadurch gekennzeichnet, dass** der zusätzliche chemische Filter in einer Menge von 29 Gewichts-%, bezogen auf das Gesamtgewicht der Zusammensetzung, vorliegt.

11. Verfahren zur Herstellung einer UVA/UVB-Zusammensetzung mit hohem Schutzfaktor, wie in irgendeinem der Ansprüche 1 bis 10 definiert, **gekennzeichnet durch** das Umfassen folgender Schritte:
(i) Auflösen von Triethanolamin in alkoholischem Träger und
(ii) unter Rühren Zugeben von Phenylbenzimidazolsulfonsäure zur der in Schritt (i) erhaltenen Mischung,
(iii) parallel hierzu Erhitzen der chemischen Filter auf eine Temperatur von 50 bis 70°C, vorzugsweise 60°C,
(iv) Aufheben des Erhitzens der in Schritt (iii) erhaltenen Mischung und, wenn sie sich auf einer Temperatur von 30 bis 50°C, vorzugsweise 40°C, befindet, deren Zugeben zu der in Schritt (ii) erhaltenen Mischung und
(v) Rühren.

## Revendications

1. Composition cosmétique à haute protection contre les UVA/UVB, caractérisée en qu'elle comprend :
(a) 0,5 à 7,5 % en poids d'acide phénylbenzimidazole-sulfonique ;
(b) 2,5 % en poids de triéthanolamine ;
(c) 6 % en poids d'un filtre chimique contre les UVA ;
(d) 20 à 40 % en poids d'un filtre chimique supplémentaire ;
(e) 40 à 70 % en poids d'un véhicule alcoolique ; et
(f) 2 % en poids d'un agent filmogène,
tous les pourcentages étant basés sur le poids total de la composition à haute protection contre les UVA/UVB et aucune quantité d'eau n'étant ajoutée à la composition.

2. Composition suivant la revendication 1, caractérisée ce que l'acide phénylbenzimidazolesulfonique est présent en une quantité allant de 1 à 5 %, en poids, sur la base du poids total de la composition.

3. Composition suivant la revendication 2, caractérisée ce que l'acide phénylbenzimidazolesulfonique est présent en une quantité de 3 %, en poids, sur la base du poids total de la composition.

4. Composition suivant l'une quelconque des revendications 1 à 3, caractérisée ce que le filtre chimique contre les UVA est choisi dans le groupe comprenant : le butylméthoxy-dibenzoylméthane, le diéthylaminohydroxybenzoylhexylbenzoate, la bis-éthylhexyloxyphénolméthoxyphényltriazine, le phényl-dibenzimidazoletétrasulfonate disodique, l'acide téréphtalylidène-dicamphosulfonique et leurs mélanges.

5. Composition suivant la revendication 4, caractérisée ce que le filtre chimique contre les UVA est choisi dans le groupe comprenant : le butylméthoxydibenzoylméthane, le diéthylaminohydroxybenzoylhexylbenzoate, et leur mélange.

6. Composition suivant les revendications 1 à 5, caractérisée ce que le filtre chimique supplémentaire est choisi dans le groupe comprenant : la benzophénone-3, l'octocrylène, l'homosalate, le salicylate d'éthylhexyle, le butylméthoxydibenzoylméthane, le diéthylaminohydroxy-benzoylhexylbenzoate, la bis-éthylhexyloxyphénolmethoxy-phényltriazine, le drométrizoletrisiloxane, le 4-méthyl-benzylidènecamphre, la benzophénone-4, la diéthylhexyl-butamidotriazone, le méthoxycinnamate d'éthylhexyle, l'éthyl-hexyltriazone, l'éthylhexyldiméthyl-paba, le salicylate d'homomenthyle, le p-méthoxycinnamate d'isoamyle, la polysilicone-15 et leurs mélanges.

7. Composition suivant l'une quelconque des revendications 1 à 6, caractérisée ce que le véhicule alcoolique comporte une quantité d'eau non supérieure à 4 %, en poids, sur la base du poids total dudit véhicule.

8. Composition suivant l'une quelconque des revendications 1 à 7, caractérisée ce que le véhicule alcoolique est présent dans ladite composition à haute protection contre les UVA/UVB, en une quantité allant de 50 à 55 % en poids, sur la base du poids total de la composition.

9. Composition suivant les revendications 1 à 8, caractérisée ce qu'elle comprend des constituants facultatifs choisis dans le groupe comprenant un antioxydant, un conservateur, un agent de formation de réseau microcristallin de support, un agent polymère, un agent copolymère, un modificateur sensoriel, un cosolvant, un agent dénaturant, un agent de consistance, un émollient, un agent humidifiant, un agent hydratant, un agent de contrainte et leurs mélanges.

10. Composition suivant les revendications 1 à 9, caractérisée ce que le filtre chimique supplémentaire est prsent en une quantité de 29 % en poids, sur la base du poids total de la composition.

11. Procédé pour préparer une composition à haute protection contre les UVA/UVB, telle que définie dans l'une quelconque des revendications 1 à 10, caractérisé en qu'il comprend les étapes suivantes :
(i) solubilisation de triéthanolamine dans un véhicule alcoolique ; et
(ii) sous agitation, addition d'acide phénylbenzimidazole-sulfonique au mélange obtenu dans l'étape (i) ;
(iii) en parallèle, chauffage des filtres chimiques à une température allant de 50 à 70°C, de préférence égale à 60°C ;
(iv) suppression du chauffage du mélange obtenu dans l'étape (iii) et, lorsque ce mélange est à une température allant de 30 à 50°C, de préférence égale à 40°C, addition de ce mélange au mélange obtenu dans l'étape (ii), et
(v) agitation.
